# EUROPÄISCHE PATENTSCHRIFT

(11) **EP 3 835 784 B1**
(45) Veröffentlichungstag und Bekanntmachung des Hinweises auf die Patenterteilung: **01.01.2025**
(21) Anmeldenummer: 20020556.5
(22) Anmeldetag: 24.11.2020
(51) Int. Cl.: G01N 9/36, G01N 33/00

(54) **VERFAHREN ZUR IDENTITÄTSPRÜFUNG EINES GASES**
METHOD FOR CHECKING THE IDENTITY OF A GAS
PROCÉDÉ DE VÉRIFICATION D'IDENTITÉ D'UN GAZ

(30) Priorität: 13.12.2019 DE 102019008671
(43) Veröffentlichungstag der Anmeldung: 16.06.2021
(73) Patentinhaber: Linde GmbH, 82049 Pullach (DE)
(72) Erfinder: Steinke, Jörg, 12557 Berlin (DE); Kaltenegger, Johann, 85051 Ingolstadt (DE)
(74) Vertreter: Gellner, Bernd

(56) Entgegenhaltungen:
- DE-A1- 102010 034 654
- US-A1- 2014 129 038

## Beschreibung

Die vorliegende Erfindung betrifft ein Verfahren und eine Anordnung zur Identitätsprüfung eines medizinischen oder pharmazeutischen Gases oder Lebensmittelgases, insbesondere von Stickstoff, gemäß den jeweiligen Oberbegriffen der unabhängigen Patentansprüche.

### Stand der Technik

In der Pharmazie und Medizin werden unterschiedliche Gase verwendet. Ein Teil solcher Gase umfasst beispielsweise die bekannten Luftgase, die insbesondere durch Tieftemperaturzerlegung von Luft oder andere Luftzerlegungsverfahren gewonnen werden können. Für eine Übersicht sei beispielsweise auf den Artikel "Medizinische Gase - Arzneimittel aus Luft" von C. und P. Imming, Pharmazeutische Zeitung 09/2013, und die einschlägigen Arzneibücher (DAB, Ph.Eur., USP) verwiesen.

Ist nachfolgend von einem "medizinischen oder pharmazeutischen Gas" die Rede, kann dieser Begriff jedes Gas bezeichnen, das selbst medizinisch oder pharmazeutisch verwendet wird, oder das zur Bereitstellung eines medizinisch oder pharmazeutisch verwendeten Gasgemischs dient. Ein solches Gas bzw. Gasgemisch kann beispielsweise als Atemgas (Sauerstoff, Stickstoff bzw. sogenannte medizinische Luft), als Gasgemisch zur Behandlung von Asthma und obstruktiven Lungenerkrankungen (beispielsweise ein Gemisch aus Sauerstoff und Helium), als Narkosegas (beispielsweise Lachgas oder eine Lachgasmischung) verwendet werden. Auch soll beispielsweise ein Hilfsgas, das in einem Syntheseverfahren zur Synthese eines Arzneimittels oder Medizinprodukts verwendet wird, oder das zur Inertisierung oder zur Bereitstellung einer Schutzatmosphäre, beispielsweise zur Verpackung eines Arzneimittels oder Medizinprodukts, dient, unter den Begriff des "medizinischen oder pharmazeutischen Gases " im hier verstandenen Sinn fallen.

Ein "Lebensmittelgas" soll im hier verstandenen Sinn ein Gas bezeichnen, das zur Herstellung oder Behandlung oder auch bei der Verpackung eines Lebensmittels, beispielsweise zur Schäumung oder Inertisierung, verwendet wird.

Die nachfolgende überwiegende Bezugnahme auf das Reingas Stickstoff soll die vorliegende Erfindung in keiner Weise einschränken. Das medizinische oder pharmazeutische Gas oder Gasgemisch oder ein entsprechendes Lebensmittelgas kann ferner in jedem beliebigen Aggregatzustand (flüssig, fest, überkritisch) vorliegen und/oder das medizinische oder pharmazeutische Gas oder ein entsprechendes Lebensmittelgas kann im Rahmen der vorliegenden Erfindung von jedem beliebigen Aggregatzustand durch geeignete Verfahren in jeden beliebigen anderen Aggregatzustand gebracht werden. Beispielsweise kann flüssiger Stickstoff im Rahmen der vorliegenden Erfindung vor seiner erfindungsgemäßen Behandlung zumindest teilweise verdampft werden, beispielsweise in aktiv oder passiv beheizten Verdampfungseinrichtungen.

Generell soll, wenn hier ein bestimmtes Gas wie Stickstoff explizit genannt ist, dies jeweils auch Gasgemische einschließen, die reich an dem entsprechenden Gas sind. Ist hier also beispielsweise von Stickstoff die Rede, kann dies auch ein an Stickstoff reiches Gasgemisch einschließen. Der Begriff "reich" soll dabei einen Gehalt von mehr als 90 vol.-%, mehr als 95 vol.-%, mehr als 99 vol.-%, mehr als 99,5 vol.-% oder mehr als 99,9 vol.-% bezeichnen. Insbesondere kann es sich jeweils um ein sogenanntes technisches Gas handeln.

Medizinisch oder pharmazeutisch verwendete Substanzen, d.h. auch entsprechende Gase, müssen typischerweise vor ihrer Verwendung zumindest einer sogenannten Identitätsprüfung unterworfen werden, um Verwechslungen auszuschließen. In der Lebensmittelindustrie ist eine entsprechende Identitätsüberprüfung ebenfalls besonders wünschenswert. Die vorliegende Erfindung stellt sich die Aufgabe, die Identitätsprüfung medizinischer Gase im oben erläuterten Sinn zu verbessern.

DE 10 2010 034 654 A1 offenbart ein Verfahren zur Identitätsüberprüfung einer in einen Tank einzufüllenden Flüssigkeit, wobei eine Testmenge der Flüssigkeit in eine Analyseeinrichtung überführt wird. In der Analyseeinrichtung wird durch eine Dichtemessung ein der Dichte der Testmenge der Flüssigkeit entsprechender Wert ermittelt. Ebenso wird in der Analyseeinrichtung die Identitätsprüfung als erfolgreich eingestuft, wenn der der Dichte in der Testmenge der Flüssigkeit entsprechende Wert als einem Sollwert entsprechend bestimmt wird, und wird die Identitätsprüfung als nicht erfolgreich eingestuft, wenn der der Dichte in der Testmenge der Flüssigkeit entsprechende Wert als dem Sollwert nicht entsprechend bestimmt wird.

### Offenbarung der Erfindung

Vor diesem Hintergrund schlägt die vorliegende Erfindung ein Verfahren zur Identitätsprüfung eines medizinischen oder pharmazeutischen Gases oder eines Lebensmittelgases gemäß Anspruch 1 vor. Ausgestaltungen sind Gegenstand der abhängigen Patentansprüche sowie der nachfolgenden Beschreibung.

Insgesamt schlägt die vorliegende Erfindung ein Verfahren gemäß Anspruch 1 zur Identitätsüberprüfung eines medizinischen oder pharmazeutischen Gases oder eines Lebensmittelgases vor, bei dem einem Gasvolumen eine Testmenge des medizinischen oder pharmazeutischen Gases oder Gasgemischs oder des Lebensmittelgases entnommen und in eine Analyseeinrichtung überführt wird. Erfindungsgemäß ist vorgesehen, dass in der Analyseeinrichtung durch eine Dichtemessung ein einer Gasdichte in der Testmenge des medizinischen oder pharmazeutischen Gases oder Lebensmittelgases entsprechender Wert ermittelt und die Identitätsprüfung als erfolgreich eingestuft wird, wenn der der Gasdichte in der Testmenge des medizinischen oder pharmazeutischen Gases oder Lebensmittelgases entsprechende Wert als einem Sollwert entsprechend bestimmt wird, und bei dem die Identitätsprüfung als nicht erfolgreich eingestuft wird, wenn der der Gasdichte in der Testmenge des medizinischen oder pharmazeutischen Gases oder Lebensmittelgases entsprechende Wert als dem Sollwert nicht entsprechend bestimmt wird.

Die vorliegende Erfindung überwindet insbesondere die Nachteile derzeit eingesetzter Verfahren zur Eingangsprüfung auf Identität entsprechender Gase, zu der die pharmazeutische Industrie auch bei Hilfsstoffen wie beispielsweise Inertisierungsgase für Arzneimittelverpackungen, verpflichtet ist, und die in der Lebensmittelindustrie besonders vorteilhaft sein könnten.

Herkömmlicherweise entnimmt das Laborpersonal beispielsweise zur Identitätsprüfung für medizinischen oder pharmazeutischen Stickstoff bei der Anlieferung mittels eines Tanklastwagens mittels einer Kannenfüllvorrichtung Flüssigstickstoff aus der Betankungsleitung des Tanklastwagens und führt zumindest einen Glimmspantest durch. Ein Glimmspantest beruht darauf, dass ein glimmender Holzspan in einer Stickstoffatmosphäre verlöscht. Der Glimmspantest ist weder spezifisch (gleiche Ergebnisse würden beispielsweise Kohlendioxid oder Argon liefern), noch automatisierbar (weil er insbesondere eine visuelle Überprüfung erfordert). Außerdem bergen die Probennahme und der interne Transport von flüssigem Stickstoff diverse Sicherheitsprobleme. Entsprechende Sicherheitsprobleme ergeben sich auch bei anderen Analyseverfahren wie der Gaschromatographie mit Wärmeleitfähigkeitsdetektion, wie sie ebenfalls teilweise derzeit eingesetzt wird, die aber eine lange Wartezeit bis zum Erhalt des Ergebnisses erfordert.

Durch die vorliegende Erfindung erübrigt sich insbesondere die erläuterte händische Probennahme und durch eine verkürzte Analytik wird die Wartezeit, beispielsweise für einen Fahrer eines Tanklastzugs. Durch den erfindungsgemäß vorgeschlagenen automatisierten Prozess lässt sich eine Analyse direkt über eine Messleitung eines Tanklastwagens in unter fünf Minuten durchführen. Dies entlastet das Laborpersonal des Kunden, weil die Durchführung der Identitätsprüfung ohne hochqualifiziertes Personal durchgeführt werden kann.

Im Rahmen der vorliegenden Erfindung lassen sich Sicherheitsprobleme beim Umgang mit tiefkalten Gasen ausräumen und die Dokumentation wird beispielsweise durch ein gedrucktes oder ein revisionssicher gespeichertes Protokoll verbessert.

Die Gasdichtebestimmung zur Identitätsfeststellung ist dabei auch spezifischer als die im in diversen Arzneibüchern vorgeschriebene Wärmeleitfähigkeitsmessung, die als Alternative zu den erläuterten Verfahren routinemäßig eingesetzt wird.

Die verwendete Messeinrichtung für das medizinische oder pharmazeutische Gas oder Lebensmittelgas kann dabei in der Nähe eines Kundentanks installiert werden. Erfindungsgemäß erfolgt die Prüfung des Inhalts eines anliefernden Tanklastwagens über dessen vorhandenen Messgasanschluss. Ein Kalibriergas kann zur regelmäßigen Selbstüberprüfung vor bzw. nach der Messung vorgesehen sein bzw. verwendet werden. Erfindungsgemäß wird nach einem entsprechenden Freispülen der Messeinrichtung dann das Probegas analysiert.

Insbesondere kann im Rahmen der vorliegenden Erfindung der erwähnte, der Gasdichte in der Testmenge des medizinischen oder pharmazeutischen Gases oder Lebensmittelgases entsprechende Wert als dem Sollwert entsprechend bestimmt werden, wenn dieser um weniger als einen vorbestimmten Schwellwert von dem Sollwert abweicht, und der der Gasdichte in der Testmenge entsprechende Wert kann als dem Sollwert nicht entsprechend bestimmt wird, wenn er um mehr als einen vorbestimmten Schwellwert von dem Sollwert abweicht.

Wie erwähnt, kann die vorliegende Erfindung bei allen medizinischen bzw. pharmazeutischen Gasen und Lebensmittelgasen eingesetzt werden, so dass hier ein Gas, das zumindest eine der Komponenten Stickstoff, Sauerstoff, Argon, Helium, Lachgas und Kohlenstoffdioxid enthält, als das medizinische oder pharmazeutisch Gas oder Lebensmittelgas der Identitätsprüfung unterworfen werden kann.

Mit besonderem Vorteil wird im Rahmen der vorliegenden Erfindung das Gasvolumen in einem Tank eines Tanklastwagens bereitgestellt. Die vorliegende Erfindung ermöglicht in diesem Zusammenhang eine besonders rasche Vor-Ort-Überprüfung des medizinischen oder pharmazeutischen Gases oder Lebensmittelgases und ggf. eine sofortige Zurückweisung fehlerhafter oder nicht spezifikationsgerechter Lieferungen.

Die Testmenge des medizinischen oder pharmazeutischen Gases oder Lebensmittelgases im Rahmen der vorliegenden Erfindung wird unter Verwendung eines Messgasanschlusses dem Versorgungstank des Tanklastwagens entnommen. Auf diese Weise lässt sich eine entsprechende, ggf. bereits vorhandene Einrichtung an einem Tanklastwagen auch für die vorliegende Erfindung vorteilhaft nutzen.

Gemäß einer bevorzugten Ausgestaltung der vorliegenden Erfindung wird ein Freigabesignal bereitgestellt, wenn die Identitätsprüfung des medizinischen oder pharmazeutischen Gases oder Lebensmittelgases als erfolgreich eingestuft wird. Dagegen kann insbesondere ein Alarmsignal, beispielsweise ein visueller Alarm an einen Fahrer des Tanklastwagens, bereitgestellt werden, wenn die Identitätsprüfung des medizinischen oder pharmazeutischen Gases oder Lebensmittelgases als nicht erfolgreich eingestuft wird, d.h. keine Identität festgestellt wurde.

Mit besonderem Vorteil kann auf Grundlage eines entsprechenden Signals eine zuvor gesperrte Abtankeinrichtung des Tanklastwagens, beispielsweise eine kryogene Pumpe oder ein elektrisch angesteuertes Ventil zur Weiterleitung des medizinischen oder pharmazeutischen Gases oder Lebensmittelgases, auf Grundlage des Freigabesignals freigegeben und damit beispielsweise das untersuchte Gas in einen Kundenspeichertank überführt werden. Wenn die Identitätsprüfung des medizinischen oder pharmazeutischen Gases oder Lebensmittelgases als nicht erfolgreich eingestuft wird, d.h. keine Identität festgestellt wurde, kann die Freigabe der Abtankeinrichtung dagegen verhindert werden. Mit besonderem Vorteil kann die Freigabe der Abtankeinrichtung manipulationssicher ausgestaltet bzw. der Versuch einer unberechtigten Freigabe entsprechend protokolliert werden.

Im Rahmen der vorliegenden Erfindung kann vorteilhafterweise der Sollwert unter Verwendung eines Kalibriergases ermittelt wird, das vor und/oder nach der Dichtemessung der Testmenge einer Dichtemessung unterworfen wird. Das Kalibriergas entspricht in seiner Zusammensetzung dem zu beprobenden medizinischen oder pharmazeutischen Gas oder Lebensmittelgas. Das Kalibriergas kann insbesondere in einem entsprechenden, durch den Gaslieferanten bereitgestellten und werksseitig identitäts- und reinheitsgeprüften Speicherbehälter bereitgestellt werden. Es kann auch vorgesehen sein, für unterschiedliche zu untersuchende medizinische oder pharmazeutische Gase oder Lebensmittelgase unterschiedliche Kalibriergase in jeweiligen getrennten und wie erläutert ausgebildeten Behältern bereitzustellen und bei Bedarf der Messeinrichtung zuzuführen.

Das erfindungsgemäße Verfahren kann umfassen, dass die Ergebnisse der Identitätsprüfung des medizinischen oder pharmazeutischen Gases oder Lebensmittelgases revisionssicher gespeichert werden. Auf diese Weise wird das erfindungsgemäße Verfahren beispielsweise auditsicher und die Ergebnisse zurückliegender Identitätsprüfungen von entsprechenden medizinischen oder pharmazeutischen Gasen oder Lebensmittelgasen können jederzeit nachvollzogen werden.

Im Rahmen der vorliegenden Erfindung eignen sich grundsätzlich alle bekannten Verfahren für die erfindungsgemäß vorgesehene Dichtemessung.

Die Erfindung erstreckt sich auch auf eine Anordnung zur Identitätsprüfung eines medizinischen oder pharmazeutischen Gases oder Lebensmittelgases, die eine Analyseeinrichtung aufweist, die zur Speisung mit einer einem Gasvolumen entnommenen Testmenge Identitätsprüfung des medizinischen oder pharmazeutischen Gases oder Lebensmittelgases eingerichtet ist. Eine derartige Anordnung ist aber nicht beansprucht.

Die Analyseeinrichtung ist dafür eingerichtet, durch eine Dichtemessung einen eine Gasdichte in der Testmenge des medizinischen oder pharmazeutischen Gases oder Lebensmittelgases entsprechenden Wert zu ermitteln und die Identitätsprüfung als erfolgreich einzustufen, wenn der der Gasdichte in der Testmenge des medizinischen oder pharmazeutischen Gases oder Lebensmittelgases entsprechende Wert als einem Sollwert entsprechend bestimmt wird, und die Identitätsprüfung anderenfalls als nicht erfolgreich einzustufen.

Zu weiteren Merkmalen einer entsprechenden Anordnung zur Identitätsprüfung eines medizinischen oder pharmazeutischen Gases oder Lebensmittelgases, die vorteilhafterweise zur Durchführung eines Verfahrens, wie es zuvor in einer Vielzahl von unterschiedlichen Ausgestaltungen beschrieben wurde, eingerichtet ist, sei auf die Erläuterungen betreffend das erfindungsgemäße Verfahren und seine Ausgestaltungen ausdrücklich verwiesen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend unter Bezugnahme auf die beigefügte Zeichnung näher erläutert, die eine Ausgestaltung der vorliegenden Erfindung veranschaulichen.

### Figurenbeschreibung

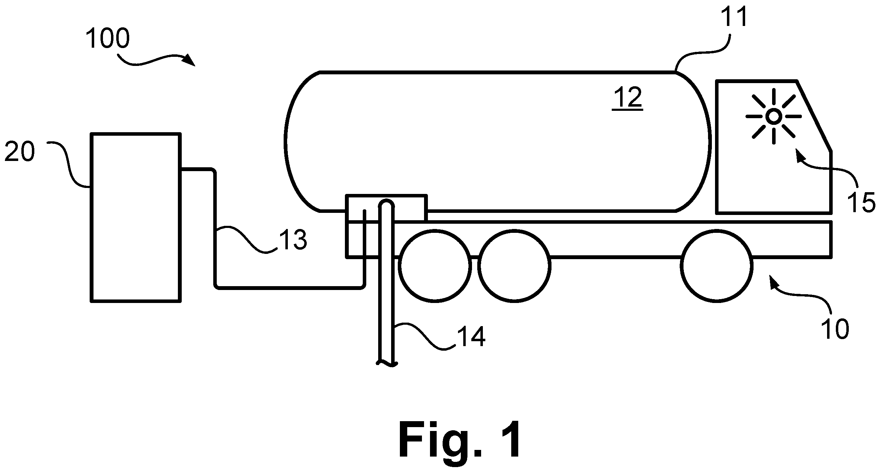

In Figur 1 ist eine Anordnung zur Durchführung der erfindungsgemäßen Identitätsüberprüfung eines medizinischen oder pharmazeutischen Gases oder Lebensmittelgases dargestellt. Die Anordnung ist dabei insgesamt mit dem Bezugszeichen 100 bezeichnet.

Aus einem Gasvolumen des medizinischen oder pharmazeutischen Gases oder Lebensmittelgases 12, das hier in einem Tank 11 eines Tanklastwagens 10 bereitgestellt wird, wird mittels eines Messgasanschlusses 13 eine Testmenge des medizinischen oder pharmazeutischen Gases oder Lebensmittelgases entnommen und in eine Analyseeinrichtung 20 überführt. Die Analyseeinrichtung 20 ist dafür eingerichtet, durch eine Dichtemessung einen einer Gasdichte in der Testmenge des medizinischen oder pharmazeutischen Gases oder Lebensmittelgases entsprechenden Wert zu ermitteln.

Die Analyseeinrichtung 20 ist ferner dafür eingerichtet, die Identitätsprüfung als erfolgreich einzustufen, wenn der der Gasdichte in der Testmenge des medizinischen oder pharmazeutischen Gases oder Lebensmittelgases entsprechende Wert als einem Sollwert entsprechend bestimmt wird, bzw. die Identitätsprüfung als nicht erfolgreich einzustufen, wenn der der Gasdichte in der Testmenge des medizinischen oder pharmazeutischen Gases oder Lebensmittelgases entsprechende Wert als dem Sollwert nicht entsprechend bestimmt wird.

Auf dieser Grundlage kann beispielsweise ein Freigabesignal bereitgestellt und mittels einer visuellen Anzeige 15, insbesondere in Form eines Ja-/Nein-Signals, einem Fahrer des Tanklastwagens 10 oder anderem Bedienpersonal angezeigt werden, wenn die Identitätsprüfung als erfolgreich eingestuft wird. Entsprechend kann mittels der visuellen Anzeige auch ein Alarmsignal bereitgestellt werden, wenn die Identitätsprüfung als nicht erfolgreich eingestuft wird. Auf Grundlage eines entsprechenden Freigabesignals kann beispielsweise auch ein eine Abtankeinrichtung 14 des Tanklastwagens freigegeben werden.

Die Analyseeinrichtung 20 kann beispielsweise einen Drucker und eine revisionssichere Speichereinrichtung aufweisen, in der ein Ergebnis der Identitätsüberprüfung revisionssicher gespeichert werden kann.

## Patentansprüche

1. Verfahren zur Identitätsüberprüfung eines medizinischen oder pharmazeutischen Gases oder Lebensmittelgases, bei dem einem Gasvolumen (12) des medizinischen oder pharmazeutischen Gases oder Lebensmittelgases eine Testmenge des medizinischen oder pharmazeutischen Gases oder Lebensmittelgases einem Versorgungstank (11) entnommen und in eine Analyseeinrichtung (20) überführt wird **wobei** dass in der Analyseeinrichtung (20) durch eine Dichtemessung ein einer Gasdichte in der Testmenge des medizinischen oder pharmazeutischen Gases oder Lebensmittelgases entsprechender Wert ermittelt wird, wobei die Identitätsprüfung als erfolgreich eingestuft wird, wenn der der Gasdichte in der Testmenge des medizinischen oder pharmazeutischen Gases oder Lebensmittelgases entsprechende Wert als einem Sollwert entsprechend bestimmt wird, und wobei die Identitätsprüfung als nicht erfolgreich eingestuft wird, wenn der der Gasdichte in der Testmenge des medizinischen oder pharmazeutischen Gases oder Lebensmittelgases entsprechende Wert als dem Sollwert nicht entsprechend bestimmt wird, **dadurch gekennzeichnet, dass** die Testmenge des medizinischen oder pharmazeutischen Gases oder Lebensmittelgases unter Verwendung eines Messgasanschlusses (13) dem Versorgungstank (11) entnommen wird und dass die Analyseeinrichtung vor der Analyse freigespült wird.

2. Verfahren nach Anspruch 1, bei dem der der Gasdichte in der Testmenge des medizinischen oder pharmazeutischen Gases oder Lebensmittelgases entsprechende Wert als dem Sollwert entsprechend bestimmt wird, wenn er um weniger als einen vorbestimmten Schwellwert von dem Sollwert abweicht, und bei dem der der Gasdichte in der Testmenge des medizinischen oder pharmazeutischen Gases oder Lebensmittelgases entsprechende Wert als dem Sollwert nicht entsprechend bestimmt wird, wenn er um mehr als einen vorbestimmten Schwellwert von dem Sollwert abweicht.

3. Verfahren nach Anspruch 1 oder 2, bei dem ein Gas, das zumindest eine der Komponenten Stickstoff, Sauerstoff, Argon, Helium, Lachgas und Kohlenstoffdioxid als das medizinische oder pharmazeutische Gas oder Lebensmittelgas der Identitätsprüfung unterworfen wird.

4. Verfahren nach einem der vorstehenden Ansprüche, bei dem das Gasvolumen des medizinischen oder pharmazeutischen Gases oder Lebensmittelgases in einem Tank (11) eines Tanklastwagens (10) bereitgestellt wird.

5. Verfahren nach einem der vorstehenden Ansprüche, bei dem ein Freigabesignal bereitgestellt wird, wenn die Identitätsprüfung als erfolgreich eingestuft wird.

6. Verfahren nach einem der Ansprüche 4 bis 5, bei dem ein Alarm- oder Sperrsignal bereitgestellt wird, wenn die Identitätsprüfung als nicht erfolgreich eingestuft wird.

7. Verfahren nach Anspruch 5, bei dem eine Abtankeinrichtung (14) des Tanklastwagens (10) auf Grundlage des Freigabesignals freigegeben wird, oder nach Anspruch 7, bei dem die Abtankeinrichtung (14) des Tanklastwagens (10) auf Grundlage des Alarm- oder Sperrsignals gesperrt wird oder bleibt.

8. Verfahren nach einem der vorstehenden Ansprüche, bei dem der Sollwert unter Verwendung eines Kalibriergases ermittelt wird, das vor und/oder nach der Dichtemessung der Testmenge einer Dichtemessung unterworfen wird.

9. Verfahren nach einem der vorstehenden Ansprüche, bei dem Ergebnisse der Identitätsprüfung revisionssicher gespeichert oder ausgedruckt werden.

## Claims

1. Method for verifying the identity of a medical or pharmaceutical gas or food gas, in which a test quantity of the medical or pharmaceutical gas or food gas is taken from a gas volume (12) of the medical or pharmaceutical gas or food gas from a supply tank (11) and transferred to an analysis device (20),
a value that corresponds to a gas density in the test quantity of the medical or pharmaceutical gas or food gas being determined in the analysis device (20) by means of a density measurement, the identity verification being classified as successful if the value that corresponds to the gas density in the test quantity of the medical or pharmaceutical gas or food gas is determined as corresponding to a target value, and the identity verification being classified as unsuccessful if the value that corresponds to the gas density in the test quantity of the medical or pharmaceutical gas or food gas is determined as not corresponding to the target value, **characterized in that** the test quantity of the medical or pharmaceutical gas or food gas is taken from the supply tank (11) using a sample gas connection (13) **and in that** the analysis device is purged prior to the analysis.

2. Method according to claim 1, in which the value that corresponds to the gas density in the test quantity of the medical or pharmaceutical gas or food gas is determined as corresponding to the target value if it deviates from the target value by less than a predetermined threshold value, and in which the value that corresponds to the gas density in the test quantity of the medical or pharmaceutical gas or food gas is determined as not corresponding to the target value if it deviates from the target value by more than a predetermined threshold value.

3. Method according to either claim 1 or claim 2, in which a gas comprising at least one of the components nitrogen, oxygen, argon, helium, nitrous oxide and carbon dioxide is subjected to the identity verification as the medical or pharmaceutical gas or food gas.

4. Method according to any of the preceding claims, in which the gas volume of the medical or pharmaceutical gas or food gas is provided in a tank (11) of a tank truck (10).

5. Method according to any of the preceding claims, in which a release signal is provided if the identity verification is classified as successful.

6. Method according to any of claims 4 to 5, in which an alarm or blocking signal is provided if the identity verification is classified as unsuccessful.

7. Method according to claim 5, in which a discharging device (14) of the tank truck (10) is released on the basis of the release signal, or according to claim 7, in which the discharging device (14) of the tank truck (10) is or remains blocked on the basis of the alarm or blocking signal.

8. Method according to any of the preceding claims, in which the target value is determined using a calibration gas which is subjected to a density measurement before and/or after the density measurement of the test quantity.

9. Method according to any of the preceding claims, in which the results of the identity verification are stored or printed out in an audit-proof manner.

## Revendications

1. Procédé permettant de vérifier l'identité d'un gaz médical ou pharmaceutique ou d'un gaz alimentaire, dans lequel une quantité de test du gaz médical ou pharmaceutique ou du gaz alimentaire est prélevée d'un volume de gaz (12) du gaz médical ou pharmaceutique ou du gaz alimentaire d'un réservoir d'alimentation (11), et transférée dans un dispositif d'analyse (20),
**dans lequel,** dans le dispositif d'analyse (20), une valeur correspondant à une densité de gaz dans la quantité de test du gaz médical ou pharmaceutique ou du gaz alimentaire est déterminée par une mesure de densité, dans lequel la vérification d'identité est considérée comme réussie si la valeur correspondant à la densité de gaz dans la quantité de test du gaz médical ou pharmaceutique ou du gaz alimentaire est déterminée comme correspondant à une valeur de consigne, et dans lequel la vérification d'identité est considérée comme non réussie si la valeur correspondant à la densité de gaz dans la quantité de test du gaz médical ou pharmaceutique ou du gaz alimentaire est déterminée comme ne correspondant pas à la valeur de consigne, **caractérisé en ce que** la quantité de test du gaz médical ou pharmaceutique ou du gaz alimentaire est prélevée du réservoir d'alimentation (11) à l'aide d'un raccord de gaz de mesure (13), **et en ce que** le dispositif d'analyse est purgé avant l'analyse.

2. Procédé selon la revendication 1, dans lequel la valeur correspondant à la densité de gaz dans la quantité de test du gaz médical ou pharmaceutique ou du gaz alimentaire est déterminée comme correspondant à la valeur de consigne lorsqu'elle s'écarte de la valeur de consigne de moins d'une valeur de seuil prédéterminée, et dans lequel la valeur correspondant à la densité de gaz dans la quantité de test du gaz médical ou pharmaceutique ou du gaz alimentaire est déterminée comme ne correspondant pas à la valeur de consigne lorsqu'elle s'écarte de la valeur de consigne de plus d'une valeur de seuil prédéterminée.

3. Procédé selon la revendication 1 ou 2, dans lequel un gaz qui contient au moins l'un des composants que sont azote, oxygène, argon, hélium, protoxyde d'azote et dioxyde de carbone, en tant que gaz médical ou pharmaceutique ou gaz alimentaire, est soumis à une vérification d'identité.

4. Procédé selon l'une des revendications précédentes, dans lequel le volume de gaz du gaz médical ou pharmaceutique ou du gaz alimentaire est fourni dans un réservoir (11) d'un camion-citerne (10).

5. Procédé selon l'une des revendications précédentes, dans lequel un signal de validation est fourni lorsque la vérification d'identité est considérée réussie.

6. Procédé selon l'une des revendications 4 à 5, dans lequel un signal d'alarme ou de blocage est fourni si la vérification d'identité est considérée non réussie.

7. Procédé selon la revendication 5, dans lequel un dispositif de déchargement (14) du camion-citerne (10) est débloqué sur la base du signal de validation, ou selon la revendication 7, dans lequel le dispositif de déchargement (14) du camion-citerne (10) est ou reste bloqué sur la base du signal d'alarme ou de blocage.

8. Procédé selon l'une des revendications précédentes, dans lequel la valeur de consigne est déterminée à l'aide d'un gaz d'étalonnage qui est soumis à une mesure de densité avant et/ou après la mesure de densité de la quantité de test.

9. Procédé selon l'une des revendications précédentes, dans lequel les résultats de la vérification d'identité sont sauvegardés ou imprimés de manière à pouvoir être révisés.
